# EUROPEAN PATENT SPECIFICATION

(11) **EP 1 206 579 B1**
(45) Date of publication and mention of the grant of the patent: **12.03.2008**
(21) Application number: 00960423.2
(22) Date of filing: 09.08.2000
(51) Int. Cl.: C12Q 1/68, G01N 33/553

(54) **PREPARATION OF METAL OXIDE SUPPORTS LOADED WITH BIOMOLECULES**
HERSTELLUNG VON METALLOXIDTRÄGERN, DIE MIT BIOMOLEKÜLEN BELADEN SIND
PREPARATION DE SUPPORTS D'OXYDES METALLIQUES CHARGES DE BIOMOLECULES

(30) Priority: 16.08.1999 EP 99202649
(43) Date of publication of application: 22.05.2002
(73) Proprietor: PamGene B.V., 5211 RX Den Bosch (NL)
(72) Inventor: VENEMA F., NL-5211 Ek's Hertogenbosch (NL)
(74) Representative: De Clercq, Ann G. Y.
(86) International application number: PCT/EP2000/007736
(87) International publication number: WO 2001/012846

(56) References cited:
- EP-A- 0 391 608
- WO-A-92/22201
- WO-A-99/02266
- BALLADUR V ET AL.: "Determination of the main forces driving DNA oligonucletide adsorption onto aminated silica wafers" JOURNAL OF COLLOID AND INTERFACE SCIENCE, vol. 194, 1997, pages 408-418, XP000870394 cited in the application
- FREY A ET AL.: "Peptomer aluminium oxide nanoparticle conjugates as systemic and mucosal vaccine candidates: Synthesis and characterization of a conjugate derived from the C4 domain of HIV-1MN Gp120" BIOCONJUGATE CHEMISTRY, vol. 8, 1997, pages 424-433, XP002128670 cited in the application
- WOJCIK S M AND PULEO D A: "Biochemical surface modification of Ti-6Al-4V for the delivery of protein to the cell-biomaterial interface" BIOMEDICAL SCIENCES INSTRUMENTATION, vol. 33, 1997, pages 166-171, XP000870316
- FADDA M B ET AL: "COVALENT COUPLING OF CONCANAVALIN A TO COMMERCIAL ALUMINA" BIOTECHNOLOGY AND APPLIED BIOCHEMISTRY,US,ACADEMIC PRESS, vol. 16, page 221-227 XP002050223 cited in the application

## Description

The invention relates to a method for the preparation of metal oxide supports loaded with biomolecules, to the thus obtained metal oxide supports, to the use of said supports for performing probe-based assays and to a kit of parts comprising said support and a detection means.

The present invention specifically relates to a method for removing non-loaded amino groups which form part of the silanating agent used to activate the metal oxide surface during the preparation of metal oxide supports loaded with biomolecules, comprising the steps of: (a) activating the surface of the support by means of an silanating agent comprising an amine group; and (b) loading the support by attaching biomolecules to the activated surface.
Such a method is known, e.g. from WO 99/002266, in which method aluminium oxide membranes are activated using 3-aminopropyl triethoxysilane (APS) after which oligonucleotide probes are covalently coupled to the activated membranes. A similar method is described for aluminiumoxide nanoparticles carrying antigens (Bioconjugate Chem. 1997, 8, 424-433). Further, for chromatographic purposes, aluminium oxide supports were used for covalent immobilization of concavalin A, after previous activation with APS (Biotechnology and Applied Biochemistry 16, 221-227 (1992)). Balladur et al., J. of Colloid and Interface Science, 194, 408-418, 1997 describe the adsorption of oligonucleotides on aminopropyl silane-modified silica wafers. These and similarly loaded metal oxide supports are thus useful for instance in probe-based assays, further for carrying biomolecules, e.g. for use as vaccines, and for separating other substances from mixtures by hybridizing, binding or interacting otherwise with those other substances.
A disadvantage of such supports is that a number of amino-groups of the silanating agent comprising an amine group used for the activation of the metal oxide surface, are still present as unloaded amino-groups even after the support has been loaded with the appropriate biomolecule. This may result in unwanted interactions (non-specific or α-specific interactions) of these amino-groups with various substances present in the medium in which the loaded support is used. For example, when used in probe-based assays, such a-specific interactions may generate high background signals disturbing the signals of the analyte, bound to the capture-biomulecules.

It has now been found that when the method of the invention is applied, the non-loaded amino-groups are removed selectively from the surface of the further loaded support without affecting the loaded part of the surface.
The invention thus relates a method for removing non-loaded amino groups which from part of the silanating agent used to activate the metal oxide surface during the preparation of metal oxide supports loaded with biomolecules, comprising the steps of: (a) activating the surface of the support by means of a silanating agent comprising an amine group; (b) loading the support by attaching biomolecules to the activated surface, and (c) treating said loaded support with an acidic solution. Such loaded silica wafers have been subject to a study regarding the effect of pH on the maximum adsorbed amount of oligonucleotides onto the aminated silica wafer [Balladur et aL, J. of Colloid and Interface Science, 194, 408-418, 1997]. Therefore this invention does not relate to (a method for the preparation of) such silica wafers.
According to another embodiment of the invention the activated and loaded support is subsequently treated with a basic solution (preferably for a prolonged period of time).
According to another embodiment of the invention the activated and loaded support is subsequently treated with a neutral solution (preferably for a prolonged period of time).

The usual methods for suppressing a-specific interactions of unloaded amino-groups on different types of supports involve chemical "capping" / "blocking" the unloaded amino-groups or prehybridizing them with DNA (see for example: Science, Vol. 270, 1995, 467-470; Nucleic Acids Research, Vol. 15 (13) 1987, 5353--5373; Nucleic Acids Research, Vol. 26 (17) 1998, 3883-3891, etc.). However, those methods are not very well defined and require more difficult procedures. Furthermore, with those methods the unloaded amino-groups are not actually removed from the surface, which is the case when the method of the present invention is applied.

The method of the invention is well defined and easy to perform and the results, e.g. in terms of signal/background ratio's, are comparable or even better when compared to the usual procedures.

In any situation in which unloaded amino-groups are to be selectively removed from metal oxide supports loaded with biomolecules, the method of the present invention is applicable, provided that a silanating agent comprising an amine group has been used to activate the metal oxide surface.

The pH value of the solution with which the loaded supports may be treated according to the invention, depends substantially on the type of biomolecule attached to the surface and on the way of attaching (loading) the biomolecules to the surface, either covalently or adsorptively.
When the biomolecule is attached to the surface covalently (i.e. they are attached to the aminogroups on the surface via some chemical linkage) and it can withstand both acidic and basic conditions, both acidic and basic solutions may be used for the removal of unloaded aminogroups. If the biomolecule is sensitive to either acidic or basic conditions, respectively a basic or acidic solution must be chosen for the removal of the unloaded amino-groups.
When adsorptively attached (i.e. the biomolecule adheres to the the surface modified with a silanating agent comprising an amine group), also the overall charge of the biomolecule is a relevant parameter. For example, when a negatively charged biomolecule is adsorptively attached to the activated surface of the metal oxide support, it may become detached at a certain pH when the charge of either the biomolecule is reduced by protonation or the charge of the surface is reduced due to deprotonation. For example, an oligonucleotide having a negatively charged backbone will be protonated at very low pH values and it will subsequently be released from the positively charged activated surface of the support. Similarly, the loaded amino-groups are deprotonated at very high pH values (e.g. 11.5) resulting in the release of the negatively charged oligonucleotide.
A person skilled in the art will know how to find the balance between effective removal of the unloaded amino-groups and the intactness of the loaded support. Some preliminary experimentation may be required to find the optimal conditions for the specific circumstances. However, use of an acidic solution is preferred, since the removal of unloaded amino-groups takes place much more rapidly than in basic conditions. At very low pH values it may even occur almost instantaneously (if the circumstances allow to use such a solution). Preferred are solutions with a pH 2 to 7. For surfaces to which oligonucleotides are attached a solution of pH 4-5 is particularly preferred.
The treatment with a neutral or basic solution is also effective, but requires longer treatment periods. The applicable pH range for a certain situation depends on the reagent used to coat the surface. The pKa of the amine groups influences the charge on the surface and thus the pH at which biomolecules, especially adsorbed biomolecules, stay attached to said surface. Of course the charge distribution on the biomolecules is also important. As explained above, depending on their negative charge, biomolecules may become detached when the pH is very low. The treatment may take several hours up to about a day, depending on the pH value of the solution, the type of metal oxide of the support and the type of reagent used to activate the surface of the support.
The acid- or base-treatment time is not very critical in terms that the performance of the loaded support is not significantly affected after long treatment periods. Also the temperature at which the method may be performed is not very critical. However, temperatures up to 40 °C are preferred. Most preferably the method is applied at room temperature (18-24°C).
Suitable (acidic or basic) solutions for use in the method according to the invention are aqueous buffered solutions adjusted to the desired pH value, for example, but not limited to, for acidic solutions: acetate buffers, phosphate buffers, citric acid buffers, and the like, for basic solutions: disodium hydrogen phosphate or sodium phosphate buffers, sodium tetraborate buffers, tris-buffers, diethanolamine/hydrogen chloride buffers, carbonate buffers, and the like. Appropriate buffers have a concentration of 40 mM, but also other concentrations may very well be applicable.

Metal oxide supports which may be used in the method of the invention are supports of metal oxides of, for example and not limited to, tantalum, titanium and aluminium, silicium as well as alloys of two or more metals and doped metals and alloys. Particularly useful supports are (electrochemically manufactured) porous metal oxide membranes. A preferred metal oxide is aluminium oxide. Preferred porous metal oxide membranes are the membranes mentioned in WO 99/02266.

The surface of the metal oxide support may be activated using different types of silanating agent comprising an amine group. Effective activating reagents are for example 3-aminopropyl triethoxysilane, 4-aminobutyl-dimethyl-methoxysilane, 3-[2-(2-aminoethylamino)ethylamino]-propyltrimethoxysilane, 3-(2-aminoethylamino)propyl-methyldimethyoxysilane, 3-(2-aminoethylamino)propyltrimethyoxysilane, 3-aminopropyl-methyl-diethoxysilane, (3-aminopropyl)-tris[2-(2-methoxyethoxy)ethoxy]silane and 4-aminobutyltriethoxysilane, however also other silanating agents comprising an amine group may be equally suitable. A preferred silanating agent is aminopropyl triethoxysilane (APS) used in an unbuffered aqueous solution. The silanation reaction may be performed in aqueous solution, in organic solution or in the gas phase.

Biomolecules may be attached to the support either covalently or adsorptively. In a preferred embodiment of the invention the biomolecules are adsorptively attached to the activated surface of the support. Preferably, the biomolecules are attached to the surface in spots, thereby forming a (micro)array of spots. A preferred method to spot the surface with biomolecules applies inkjet technology. This technology allows for the accurate deposition of defined volumes of liquid. (See e.g. T.P. Theriault: DNA diagnostic systems based on novel Chem-Jet technologies, IBC Conference on Biochip Array Technologies, Washington DC, May 10, 1995)

In a highly preferred embodiment of the invention an acidic solution of pH 5 is used for the preparation of APS-activated aluminium oxide porous membranes (which are electrochemically manufactured), spotted with arrays of oligonuceotides, which oligonucleotides are adsorptively attached to the activated surface.

Metal oxide supports prepared according to the method of the invention, are very useful for diagnostic purposes, for example in probe-based assays. In particular (micro)arrays, on the surface of which essentially no free amino groups are present that lead to α-specific binding, are very suitable for that purpose. In such arrays, the density of amino groups on the surface area between the spots is significantly lower than the density of amino groups in the spots. Preferably, the arrays comprise different biomolecules in different spots, allowing multi-analyte detection. An analyte is a substance, usually a biomolecule, in a mixture which may be detected because of its capability to interact specifically with a selected reagens (for example another biomolecule capable of reacting with the analyte) (on a support).
Probe-based assays comprise for example nucleic acid hybridization assays and immunological assays. In such assays, a sample which comprises an analyte is contacted with a loaded support prepared according to the invention. The analyte is subsequently allowed to bind to the biomolecule which is attached to the surface of the support. Detection of binding can be performed by (1) adding a detection means, for example a substance capable of binding to the analyte, which substance is provided with a label, (2) allowing the detection means to bind to the complex of the analyte and the biomolecule, and (3) determining whether the label is present at the position where the biomolecule was attached. Alternatively, the analyte may already have been provided with a label, in which case binding to the biomolecule can be detected directly, without the addition of a detection means.

Biomolecules which may be used in the process of the invention include oligonucleotides and other negatively charged capture molecules, such as antibodies, antigens, peptides, receptors, haptens and ligands for receptors (which may be modified to introduce (additional) negative charges). Preferred are oligonucleotides. However, the scope of the invention is not limited to these specific molecules. The method may very well be useful to supports loaded with other types of molecules as well.

The invention is further illustrated by the following examples.

### EXAMPLES

### Preparation of APS-membranes:

Membranes (Anodisc 25, 0.2 um; Whatman) were silanated by placing them in a 1% solution of 3-aminopropyltriethoxysilane (Acros, APS) in demineralised water (milliQ), for a period of 1 hr on a plateshaker. The membranes were soaked in water to remove the bulk of the APS molecules and subsequently washed individually by sucking 5 ml of milliQ through the membranes to remove the last traces of free APS. These membranes were heated for 2 hr's at 120 °C under vacuum. After cooling, these "APS-membranes" were stored in a vacuum exsiccator, under argon over KOH until they were used.

### Stability study of APS-membranes:

The following buffers were made:
- 40 mM Acetate buffer pH 5.0; from sodiumacetate and acetic acid
- 40 mM Acetate buffer pH 6.0; from sodiumacetate and acetic acid
- 40 mM Phosphate buffer pH 7.0; from sodiumdihydrogenphosphate and NaOH
- 40 mM Borate buffer pH 8.0; from sodium tetraborate and hydrogen chloride
- 40 mM Borate buffer pH 9.0; from sodium tetraborate and hydrogen chloride

The APS membranes were incubated at the above mentioned pH's using the appropriate buffer for a period between 0.25 and 22 hr's (see table 1, for details). After this period the membrane was removed from the buffer solution and washed with milliQ and ethanol and dried under vacuum at room temperature. Subsequently the amount of amino-groups was determined using a modified trinitrobenzenesulfonic acid (TNBS) method [Habeeb, A.F.S.A. (1996), Anal. Biochem 14, 328. See also Sashidhar, R.B., Capoor, A.K., Ramana, D., (1994), J. Immumol. M]. A commercially available stock solution (Sigma) of 5% (m/v) of TNBS was diluted 25-fold with a 50 mM borate buffer of pH 10.0 directly before the amino-group determination was performed. Each treated membrane was placed individually in a beaker and 4 ml of the diluted TNBS solution was added and the membrane was incubated on a plateshaker for 1 hr at room temperature. The yellow/orange membranes were thoroughly washed (by using vacuum to suck liquids through the membrane) with milliQ to remove excess TNBS. Subsequently the membranes were dissolved in a known amount (e.g. 4.0 ml) of a 4.0 M NaOH solution in milliQ. After filtration of this solution over a 0.45 µm filter, the absorption was measured using an UV/vis spectrophotometer. Using the extinction coefficient of the adduct at 390 nm of 11929 1/mol/cm the number of amino groups per membrane that reacted with TNBS could be determined. In table 1 the decrease (in %) in amino-groups as function of time and pH is given.

**Table 1: degradation as function of pH and time of APS membranes**

| Time (hr) | Percentage of aminogroups at | | | | |
|---|---|---|---|---|---|
| | pH 5.0 | pH 6.0 | PH 7.0 | pH 8.0 | pH 9.0 |
| Blanc exp. | 100 | 100 | 100 | 100 | 100 |
| 0.25 | 54 | | | | |
| 0.5 | 19 | | | | |
| 1 | 9 | | | | |
| 1.5 | | 49 | | | |
| 2.5 | | 17 | 82 | 96 | 51 |
| 6 | | | 69 | 52 | 34 |
| 22 | | | 54 | 42 | 21 |

From this table it is clear that the loss of aminogroups is fastest at lower pH's.

### Protective effects of loading of the activated surface of a support:

### (a) Protective effect of the TNBS group:

In another experiment the aminogroups on an APS membrane reacted with TNBS as described above. This yielded membranes that contain covalently linked chromophores that consist of the trinitrobenzene adduct of the amino group (APS-TNB membranes).

These membranes were treated with the same buffers mentioned above for a period of 22 hr's at room temperature. In table 2 the remaining amount of chromophores after this period, as determined with the above mentioned method, are summarized.

**Table 2: stability of TNB-APS membranes**

| pH applied | Resulting aminogroups cq chromophores (%) |
|---|---|
| Blanc experiment | 100 |
| 5.0 | 71 |
| 6.0 | 95 |
| 7.0 | 83 |
| 8.0 | 90 |
| 9.0 | 95 |

This table clearly shows, that functionalisation of the aminogroups results in a large increase of the stability. In other words, the majority of the functionalized aminogroups attached to the surface is not affected.

### (b) Protective effect of DNA

A haring sperm DNA solution of 1 µg/ml in milliQ was prepared and 1 µl of this solution was pipetted onto the APS membranes in spots. These membranes were devided in two sets: one which was treated with pH 5 buffer (for 1 hr) and the other which were not. Both sets of membranes were subsequently treated with TNBS (method described above) in order to detect any decrease in amino-groups. This yielded the following results. The total surface of the untreated membrane showed the expected orange colour since no degradation (removal) of APS groups had taken place. The surface of the membrane that was treated at pH 5.0 was not orange but very slightly yellow, due to the degradation of nearly all APS molecules. However, at the spots where the DNA was pipetted on the membrane, an orange colour was still visible, indicating the protective effect of the DNA on the degradation process of the aminogroups on the surface. It was shown in a blanc experiment that the amino groups of DNA did not react with TNBS in such a way that this could explain the orange colour observed for the pH 5 treated membrane.

### (c) Protective effect of oligonucleotides

A solution of 200uM oligonucleotide with the sequence 5'TAT GGC TCT CCC GGG AGGGGG GGT CCT GGA 3' was prepared. This solution was pipetted onto two unmodified membranes and onto two APS membranes. In this process only one half of every membrane was covered with the oligonucleotide. Subsequently these membranes were dried at 37 °C for one hr. One unmodified and one APS membrane were treated with pH 5 solution as described above. Subsequently all membranes were washed with phosphate buffer pH 7.4 and ethanol and dried. All membranes were placed in the diluted TNBS solution as described above and incubated for one hr. After washing and drying, the unmodified membranes were totally white, while the total surface of the APS membrane that was not treated with pH 5 was yellow.
The pH 5 treated APS membrane showed a slightly yellow color on the half that was covered with the oligonucleotides while the other half was totally white.
This experiments clearly shows the protective effect of oligonucleotides.

### (d) Arrays of oligonucleotides:

The protective effect was also visualised in a different way using hybridization of oligonucleotides:
Using a commercially available spotter (Packard instruments), droplets of 300 picoliter of oligonucleotide A (100 µM dissolved in milliQ) were spotted onto APS membranes:
This oligonucleotide A is defined by the sequence: 5'-TTG TAC AGA ACT GGA AAA GGA 3'. Also available for hybridization experiments is oligonucleotide B (sequence 5'-TCC TTT TCC ACT TCT GTA CAA 3') which is complementary to A and labeled with a fluoresceine moiety at the 5' end.

After spotting, all membranes were dried at 50 °C for 1 hr. One set of membranes was incubated in an acetate buffer (40 mM, pH 5.0) for 30 minutes and subsequently washed with milliQ, ethanol, and dried under vacuum. These membranes will be referred to as "pH 5 arrays". As a blanc experiment the other set of membranes was not treated with the pH 5.0 solution, but it was only washed with milliQ, ethanol and dried under vacuum. These membranes will be called "blanc array". All membranes were stored under argon untill being used for hybridisation experiments.

The membranes were placed in a homebuilt device that allows 25 µl of a phosphate buffer (pH 7.0) containing the complementary oligonucleotide B being flushed back and forth through the membrane. If the solution is below this device, a hybridised nucleotide on the membrane can directly be seen due to its fluorescent label using an ordinary fluorescence microscope and a CCD-camera. With this setup, the data in table 3 have been collected. One cycle is defined as the 25 µl being pumped up and down one time through the membrane. Specific signals have been corrected for the background signal.

**Table 3: Specific and a-specific interactions of pH 5 arrays and blanc arrays**

| | Signal (arb.units) for pH 5 arrays | | | Signal (arb.units) for blanc arrays | |
|---|---|---|---|---|---|
| Cycles | Specific | Background | Cycles | Specific | Background |
| 0 | 0 | 5 | 0 | 0 | 5 |
| 2 | 4 | 8 | 1 | 0 | 41 |
| 5 | 7 | 8 | 5 | 2 | 47 |
| 10 | 11 | 9 | | | |
| 15 | 13 | 9 | | | |
| 20 | 15 | 9 | | | |

From this table the following observations are important:
- The background signal for pH 5 arrays is low and nearly constant
- The specific hybridisation for pH 5 arrays takes place rapidly and can easily be monitored
- The blanc array does not show specific signals, due to a strong a-specific interaction between the amino-groups (which are present all over the membrane) and oligonucleotide B.
- The a-specific interactions (background) that are seen for the blanc arrays, is highly reduced by the acidic treatment.

Thus, this experiment shows that the acidic treatment removes all amino groups that are not covered and protected by oligonucleotides. Oligonucleotide A is not removed from the surface indicating that it is still captured by aminogroups on the surface below the nucleotides which have not been removed.

## Claims

1. A method for removing non-loaded amino groups which form part of the silanating agent used to activate the metal oxide surface during the preparation of metal oxide supports loaded with biomolecules, comprising the steps of:
(a) activating the surface of the support by means of a silanating agent comprising an amine group;
(b) loading the support by attaching biomolecules to the activated surface, and,
(c) treating said loaded support with an acidic solution.

2. A method for removing non-loaded amino groups which form part of the silanating agent used to activate the metal oxide surface during the preparation of metal oxide supports loaded with biomolecules, comprising the steps of:
(a) activating the surface of the support by means of a silanating agent comprising an amine group;
(b) loading the support by attaching biomolecules to the activated surface, and,
(c) treating said loaded support with a basic or neutral solution.

3. The method of claim 1 or 2, wherein the solution is pH 2 to 7.

4. The method of claim 3, wherein the biomolecules are oligonucleotides and the pH is 4-5.

5. The method of claim 1, wherein the metal oxide support is a (electrochemically manufactured) porous metal oxide membrane.

6. The method of claim 5, wherein the metal oxide is aluminium oxide.

7. The method of claim 1, wherein the support is activated by means of a silanating agent comprising an amine group selected from 3-aminopropyltriethoxysilane, 4-aminobutyldimethyl-methoxysilane, 3-[2-(2-aminoethylamino)ethylamino]propyl-trimethoxysilane, 3-(2-aminoethylamino)propyl-methyldimethyoxysilane, 3-(2-aminoethylamino)propyl-trimethyloxysilane, 3-aminopropyl-methyl-diethoxysilane, (3-aminopropyl)tris[2-(2-methoxyethoxy)ethoxy]silane and 4-aminobutyltriethoxysilane.

8. The method of claim 7, wherein the silanating agent comprising an amine group is 3-aminopropyltriethoxysilane.

9. The method of claim 8, wherein 3-aminopropyl triethoxysilane is used in an unbuffered aqueous solution.

10. The method of claim 1, wherein the biomolecules are adsorptively attached to the activated surface of the support.

11. The method of claim 1, wherein the biomolecules are attached to the activated surface in spots, thereby forming an array of spots.

12. The method of claim 11, wherein the biomolecules attached to the surface in different spots may be the same or different.

13. The method of claim 1, wherein the biomolecules are oligonucleotides.

## Patentansprüche

1. Verfahren zum Entfernen von nichtgeladenen Aminogruppen, die einen Teil des Silanisiermittels bilden, das zum Aktivieren der Metalloxidoberfläche während der Herstellung von Metalloxidträgern verwendet wird, die mit Biomolekülen beladen sind, umfassend die Schritte des:
(a) Aktivierens der Oberfläche des Trägers durch ein Silanisiermittel umfassend eine Amingruppe;
(b) Beladen des Trägers durch Anlagern von Biomolekülen an die aktivierte Oberfläche und
(c) Behandeln des beladenen Trägers mit einer sauren Lösung.

2. Verfahren zum Entfernen von nichtgeladenen Aminogruppen, die einen Teil des Silanisiermittels bilden, das zum Aktivieren der Metalloxidoberfläche während der Herstellung von Metalloxidträgern verwendet wird, die mit Biomolekülen beladen sind, umfassend die Schritte des:
(a) Aktivierens der Oberfläche des Trägers durch ein Silanisiermittel umfassend eine Amingruppe;
(b) Beladen des Trägers durch Anlagern von Biomolekülen an die aktivierte Oberfläche und
(c) Behandeln des beladenen Trägers mit einer basischen oder neutralen Lösung.

3. Verfahren nach Anspruch 1 oder 2, wobei die Lösung einen pH-Wert von 2 bis 7 aufweist.

4. Verfahren nach Anspruch 3, wobei die Biomoleküle Oligonucleotide sind und der pH-Wert 4 - 5 beträgt.

5. Verfahren nach Anspruch 1, wobei der Metalloxidträger eine (elektrochemisch hergestellte) poröse Metalloxidmembran ist.

6. Verfahren nach Anspruch 5, wobei das Metalloxid Aluminiumoxid ist.

7. Verfahren nach Anspruch 1, wobei der Träger durch ein Silanisiermittel aktiviert wird umfassend eine Aminogruppe ausgewählt unter 3-Aminopropyltriethoxysilan, 4-Aminobutyldimethylmethoxysilan, 3-[2-(2- Aminoethylamino)ethylamino]propyltrimethoxysilan, 3-(2-Aminoethylamino)propylmethyldimethoxysilan, 3-(2-Aminoethylamino)propyltrimethoxysilan, 3-Aminopropylmethyldimethoxysilan, (3- Aminopropyl)tris[2-(2-methoxyethoxy)ethoxy]silan und 4-Aminobutyltriethoxysilan.

8. Verfahren nach Anspruch 7, wobei das Silanisiermittel, das eine Amingruppe umfasst, 3-Aminopropyltriethoxysilan ist.

9. Verfahren nach Anspruch 8, wobei das 3-Aminopropyltriethoxysilan in einer ungepufferten wässrigen Lösung verwendet wird.

10. Verfahren nach Anspruch 1, wobei die Biomoleküle adsorptiv an die aktivierte Oberfläche des Trägers angelagert sind.

11. Verfahren nach Anspruch 1, wobei die Biomoleküle an die aktivierte Oberfläche in Fleckenform angelagert sind, wobei eine Anordnung von Flecken gebildet wird.

12. Verfahren nach Anspruch 11, wobei die Biomoleküle, die an die Oberfläche in Form verschiedener Flecken angelagert sind, gleich oder verschieden sein können.

13. Verfahren nach Anspruch 1, wobei die Biomoleküle Oligonucleotide sind.

## Revendications

1. Procédé d'élimination des groupes amino non chargés qui constituent l'agent de silanation utilisé pour activer la surface de l'oxyde métallique pendant la préparation de supports à base d'oxyde métallique chargés avec des biomolécules, comprenant les étapes consistant à :
(a) activer la surface du support au moyen d'un agent de silanation comprenant un groupe amino ;
(b) charger le support en fixant les biomolécules à la surface activée, et
(c) traiter ledit support chargé avec une solution acide.

2. Procédé d'élimination des groupes amino non chargés qui constituent l'agent de silanation utilisé pour activer la surface de l'oxyde métallique pendant la préparation de supports à base d'oxyde métallique chargés avec des biomolécules, comprenant les étapes consistant à :
(a) activer la surface du support au moyen d'un agent de silanation comprenant un groupe amino;
(b) charger le support en fixant les biomolécules à la surface activée, et
(c) traiter ledit support chargé avec une solution basique ou neutre.

3. Procédé selon la revendication 1 ou 2, dans lequel la solution a un pH de 2 à 7.

4. Procédé selon la revendication 3, dans lequel les biomolécules sont des oligonucléotides et le pH va de 4 à 5.

5. Procédé selon la revendication 1, dans lequel le support à base d'oxyde métallique est une membrane poreuse à base d'oxyde métallique (fabriquée de manière électrochimique).

6. Procédé selon la revendication 5, dans lequel l'oxyde métallique est de l'oxyde d'aluminium.

7. Procédé selon la revendication 1, dans lequel le support est activé au moyen d'un agent de silanation comprenant un groupe amino choisi parmi le 3-aminopropyltriéthoxysilane, le 4-aminobutyldiméthylméthoxysilane, le 3-[2-(2-aminoéthylamino)éthylamino]propyl-triméthoxysilane, le 3-(2-aminoéthylamino)propyl-méthyldiméthyloxysilane, le 3-(2-aminoéthylamino)propyl-triméthyloxysilane, le 3-aminopropyl-méthyldiéthoxysilane, le (3-aminopropyl)tris[2-(2-méthoxyéthoxy)éthoxy]silane et le 4-aminobutyltriéthoxysilane.

8. Procédé selon la revendication 7, dans lequel l'agent de silanation comprenant un groupe amino est le 3-aminopropyltriéthoxysilane.

9. Procédé selon la revendication 8, dans lequel le 3-aminopropyltriéthoxysilane est utilisé dans une solution aqueuse non tamponnée.

10. Procédé selon la revendication 1, dans lequel les biomolécules sont fixées de manière adsorbante à la surface activée du support.

11. Procédé selon la revendication 1, dans lequel les biomolécules sont fixées à la surface activée en plusieurs points, en formant ainsi un réseau de points.

12. Procédé selon la revendication 11, dans lequel les biomolécules fixées à la surface en différents points peuvent être identiques ou différentes.

13. Procédé selon la revendication 1, dans lequel les biomolécules sont des oligonucléotides.
